# EUROPÄISCHE PATENTSCHRIFT

(11) **EP 0 741 687 B1**
(45) Veröffentlichungstag und Bekanntmachung des Hinweises auf die Patenterteilung: **12.08.1998**
(21) Anmeldenummer: 94905714.5
(22) Anmeldetag: 27.01.1994
(51) Int. Cl.: C07C 59/62, A61K 31/557, C07C 59/72, C07C 69/16, C07D 309/30

(54) **NEUE LEUKOTRIEN-B4-DERIVATE, VERFAHREN ZU IHRER HERSTELLUNG UND IHRE VERWENDUNG ALS ARZNEIMITTEL**
LEUKOTRIENE B4 DERIVATIVES, METHODS OF PREPARING THEM AND THEIR USE AS DRUGS
NOUVEAUX DERIVES DES LEUCOTRIENES B4, LEURS PROCEDES DE FABRICATION ET LEUR UTILISATION COMME MEDICAMENTS

(43) Veröffentlichungstag der Anmeldung: 13.11.1996
(73) Patentinhaber: SCHERING AKTIENGESELLSCHAFT, 13342 Berlin (DE)
(72) Erfinder: SKUBALLA, Werner, D-13465 Berlin (DE); BUCHMANN, Bernd, D-10557 Berlin (DE); HEINDL, Josef, D-14129 Berlin (DE); FRÖHLICH, Wolfgang, D-10709 Berlin (DE); EKERDT, Roland, D-13369 Berlin (DE); GIESEN, Claudia, D-13587 Berlin (DE)
(86) Internationale Anmeldenummer: EP9400216
(87) Internationale Veröffentlichungsnummer: WO9520564

(56) Entgegenhaltungen:
- Keine einschlägigen Dokumente gefunden

## Beschreibung

Die Erfindung betrifft neue Leukotrien B₄-Derivate, Verfahren zu ihrer Herstellung sowie ihre Verwendung als Arzneimittel. Die neuen Verbindungen sind optisch aktive Struktranaloge von vorbekannten Leukotrien-B₄-Antagonisten, die einen Sechsring als Grundstrukturelement enthalten (DE-A 39 17 597, DE-A 42 27 790.6). Leukotrien B₄ (LTB₄) wurde von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

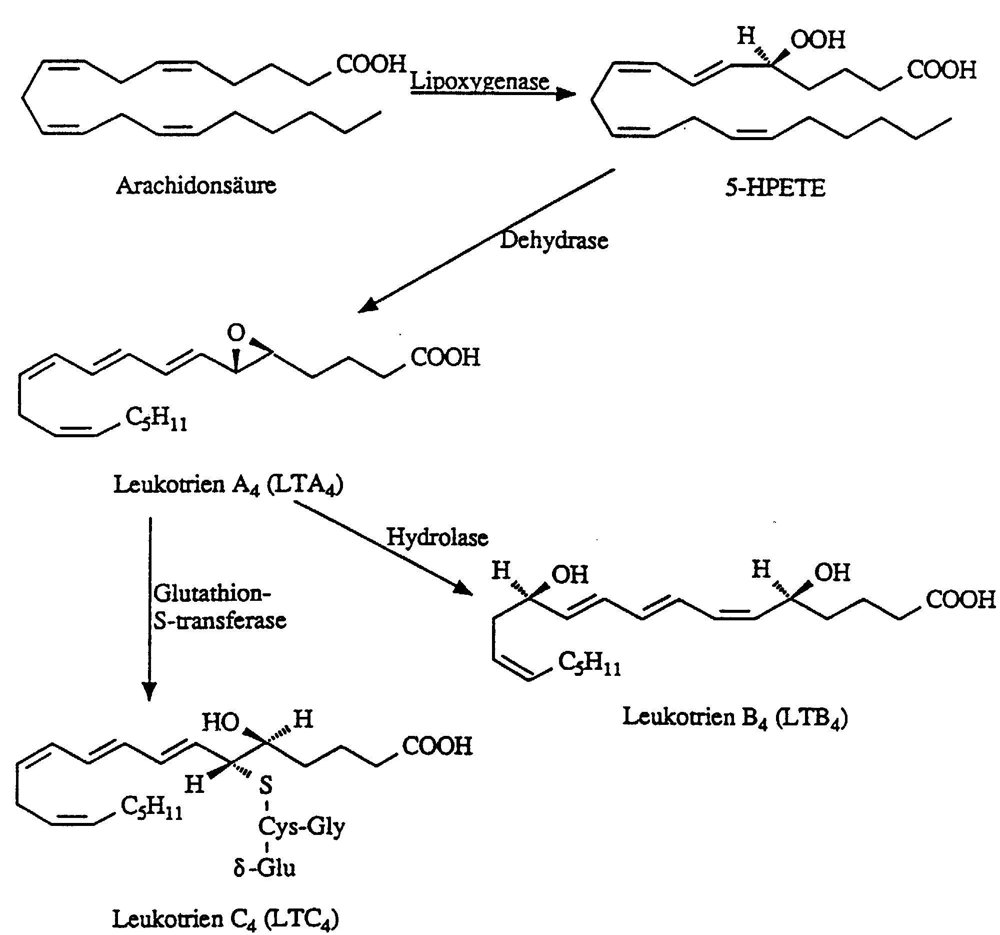

Die Nomenklatur der Leukotriene kann folgenden Arbeiten entnommen werden:
a) B. Samuelsson et al., Prostaglandins 19 645 (1980); 17, 785 (1979).
b) C. N. Serhan et al., Prostaglandins 34, 201 (1987)

Die physiologische und insbesondere die pathophysiologische Bedeutung des Leukotrien B₄ wird in einigen neueren Arbeiten zusammengefaßt: a) The Leukotriens, Chemistry and Biology eds. L. W. Chakrin, D. M. Bailey, Academic Press 1984. b) J. W. Gillard et al., Drugs of the Future 12, 453(1987). c) B. Samuelson ., Sciences 237, 1171 (1987). d) C W. Parker, Drug Development Research 10, 277 (1987). Hieraus ergibt sich, daß LTB₄ ein wichtiger Entzundungsmediator füe entzündliche Erkrankungen ist, bei denen Leukozyten in das erkrankte Gewebe einwandern.
Die Effekte von LTB₄ werden auf zellulärer Ebene durch die Bindung von LTB₄ an einen spezifischen Rezeptor ausgelöst.
Von LTB₄ weiß man, daß es die Adhäsion von Leukozyten an die Blutgefäßwand verursacht. LTB₄ ist chemotaktisch wirksam, d. h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatiden entweder ursächlich beteiligt, oder es besteht ein Zusammenhang zwischen der Persistenz der Dermatiden und den Leukotrienen. Deutlich erhöhte Leukotrienkonzentrationen wurden beispielsweise in der Haut von Patienten mit Psoriasis oder atopischer Dermatitis gemessen.
Leukotriene und insbesondere LTB₄ sind auch an Erkrankungen innerer Organe beteiligt, für die eine akute oder chronische entzündliche Komponente beschrieben wurde, z. B.: Gelenkerkrankungen (Athritis); Erkrankungen des Respirationstraktes (Asthma, Rhinitis und Allergien); entzündliche Darmerkrankungen (Colitis); sowie Reperfusionsschäden (an Herz-, Darm- oder Nierengewebe), die durch zeitweisen krankhaften Verschluß von Blutgefäßen entstehen.
Weiterhin sind Leukotriene und insbesondere LTB₄ bei der Erkrankung an multipler Sklerose beteiligt und bei dem klinischen Erscheinungsbild des Schocks (ausgelöst durch Infektionen, Verbrennungen oder bei Komplikationen bei der Nierendialyse oder anderen extra besprochenen Perfusionstechniken).
Leukotriene und insbesondere LTB₄ haben weiterhin einen Einfluß auf die Bildung von weißen Blutkörperchen im Knochenmark, auf das Wachstum von glatten Muskelzellen, vom Keratinozyten und von B-Lymphozyten. LTB₄ ist daher bei Erkrankungen mit entzündlichen Prozessen und bei Erkrankungen mit pathologisch gesteigerter Bildung und Wachstum von Zellen beteiligt.
Erkrankungen mit diesem Erscheinungsbild stellen z. B. Leukernie oder Artherosklerose dar.

Durch die Antagonisierung der Effekte insbesondere von LTB₄ sind die Wirkstoffe und deren Darreichungsformen dieser Erfindung spezifische Heilmittel bei der Erkrankung von Mensch und Tier, bei denen insbesondere Leukotriene eine pathologische Rolle spielen.

Neben therapeutischen Möglichkeiten, die sich aus einer Antagonisierung der LTB₄-Wirkung mit LTB₄-Analoga ableiten lassen, konnte auch die Nützlichkeit und potentielle Anwendung von Leukotrien B₄-Agonisten zur Behandlung von Pilzerkrankungen der Haut gezeigt werden (H. Katayama, Prostaglandins 34, 797 (1988)).

Die Erfindung betrifft Leukotrien-B₄-Derivate der allgemeinen Formel I, 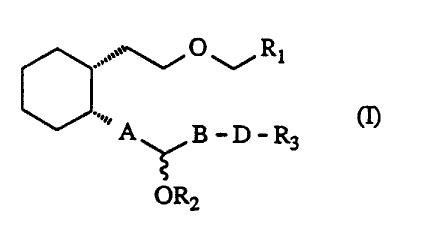 worin
- R₁: CH₂OH, CH₃, CF₃, COOR₄, CONR₅R₆,
- R₂: H oder ein organischer Säurerest mit 1-15 C-Atomen darstellt,
- R₃: H, gegebenenfalls einfach oder mehrfach substituiertes C₁-C₁₄-Alkyl, C₃-C₁₀-Cycloalkyl, gegebenenfalls unabhängig voneinander einfach oder mehrfach durch Halogen, Phenyl, C₁-C₄-Alkyl, C₁-C₄-Alkoxy, Fluormethyl, Chlormethyl, Trifluormethyl, Carbonyl, Carboxyl oder Hydroxy substituierter C₆-C₁₀-Arylrest oder ein 5-6 gliedriger aromatischer heterocyclischer Ring mit wenigstens 1 Heteroatom symbolisieren,
- R₄: Wasserstoff, C₁-C₁₀ Alkyl, C₃-C₁₀ Cycloalkyl, gegebenenfalls durch 1-3 Halogen, Phenyl, C₁-C₄-Alkyl, C₁-C₄-Alkoxy, Fluormethyl, Chlormethyl, Trifluormethyl, Carboxyl oder Hydroxy substituierter C₆-C₁₀-Arylrest, CH₂-CO(C₆-C₁₀) Aryl oder ein 5-6 gliedriger Ring mit wenigstens 1 Heteroatom bedeutet,
- A: eine Trans, trans-CH=-CH-CH=-CH, eine -CH₂CH₂-CH=CH- oder eine Tetramethylengruppe,
- B: eine C₁-C₁₀- gerad- oder verzweigtkettige Alkylengruppe, die gegebenenfalls durch Fluor substituiert sein kann oder die Gruppe 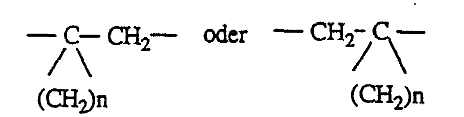 symbolisiert,
- D: eine Direktbindung, Sauerstoff, Schwefel, -C≡C-, -CH=CR₇, oder gemeinsam mit
- B: auch eine Direktbindung bedeuten können,
- R₅ und R₆: gleich oder verschieden sind und H oder C₁-C₄-Alkyl oder R₆ H und R₅ C₁-C₁₅-Alkanoyl oder R₈SO₂- darstellen, gegebenenfalls mit OH substituiert sind,
- R₇: H, C₁-C₅ Alkyl, Chlor, Brom bedeutet,
- R₈: die gleiche Bedeutung wie R₃ besitzt,
- n: 2-5 ist sowie, wenn R₄ Wasserstoff bedeutet, deren Salze mit physiologisch verträglichen Basen und deren Cyclodextrinclathrate.

Die Gruppe OR₂ kann α- oder β-ständig sein. Die Formel I umfaßt sowohl Racemate als auch die möglichen reinen Diastereomeren und Enantiomeren.

Als Allylgruppen R₄ kommem gerad- oder verzweigtkettige Alkylgruppen mit 1-10 C-Atomen in Betracht, wie beispielsweise Methyl, Ethyl, Propyl, Butyl, Isobutyl, tert-Butyl, Penty, Neopentyl, Hexyl, Heptyl, Decyl.
Die Alkylgruppen R₄ können gegbenenfalls ein- bis mehrfach substituiert sein durch Halogenatome, Alkoxygruppen, gegebenenfalls substituierte Aryl- bzw. Aroylgruppen mit 6-10 C-Atomen (Bezüglich möglicher Substituenten siehe unter Aryl R₄), Dialkylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₄ sind solche mit 1-4 C-Atomen zu nennen.

Die Cycloalkylgruppe R₄ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl, Cyclohexyl, Methylcyclohexyl.

Als Arylgruppen R₄ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, eine Fluormethyl-, Trifluormethyl-, Carboxyl-, Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Als heterocyclische Gruppen R₄ kommen 5- und 6-gliedrige aromatische Heterocyclen in Frage, die wenigsten 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl u.a.

Als Säurerest R₅ kommen solche physiologisch verträglicher Säuren in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt. Beispielweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als bevorzugte Arylsulfonylreste und Alkansulfonylreste R₈SO₂- werden solche betrachtet, die sich von einer Sulfonsäure mit bis zu 10 Kohlenstoffatomen ableiten. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)aminosulfonsäure, N,N-Diisobutylaminosulfonsäure, N,N-Dibutylaminosulfonsäure, Pyrrolidino-, Piperidino-, Piperazino-, M-Methylpiperazino- und Morpholinosulfonsäure in Frage.

Als Alkylgruppen R₃ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl R₅) substituiert sein können. Beispielsweise seien genannt Methy-, Ethyl-, Propyl-, Butyl-, Isobutyl-, tert-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R₃ Halogen-substituiert, kommen als Halogene Fluor, Chlor und Brom in Frage.

Als Beispiele für Halogen-substituierte Alkylgruppen R₃ kommen Alkyle mit terminalen Trifluormethylgruppen in Betracht.

Die Cycloalkylgruppe R₃ kann im Ring 3-10, vorzugsweise 3-6 Kohlenstoffatome enthalten. Die Ringe können durch Alkylgruppen mit 1-4 Kohlenstoffatomen gegebenenfalls durch Halogene substituiert sein. Beispielsweise genannt seien Cyclopropyl, Cyclobutyl, Cyclopentyl, Cyclohexyl, Methyl-cyclohexyl, Fluor-cyclohexyl.

Als substituierte bzw. unsubstituierte Arylgruppen R₃ kommen beispielweise in Betracht: Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen eine Chlormethyl-, Fluormethyl-, Trifluormethyl-, Carboxyl-, C₁-C₄ Alkoxy- oder Hydroxygruppe. Bevorzugt ist die Substitution in 3- und 4- Stellung an Phenylring zum Beispiel durch Fluor, Chlor, Alkoxy oder Trifluormethyl oder in 4-Stellung durch Hydroxy.

Als heterocyclische aromatische Gruppen R₃ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3- Furyl, 3-Thienyl, u.a.

Als Alkylengruppe B kommen geradkettige oder verzweigte, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1,2-Difluorethylen, 1-Fluorethylen, 1-Methyltetramethylen, 1-Methyl-tri-methylen, 1-Methylen-ethylen, 1-Methylen-tetramethylen.
Die Alkylengruppe B kann weiterhin die Gruppe 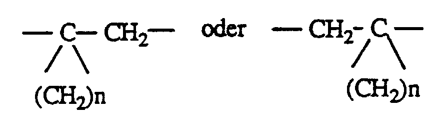 darstellen, wobei n=2-5, bevorzugt 3-5 bedeutet.

Als Säurereste R₂ kommen solche von physiologisch verträglichen Säureresten in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alky, Hydroxy, C₁₋₄-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert-Butylessigsäure, Cyclopentylessigsäure, Cyclohexylessigsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als bevorzugte Säurereste R₂ und R₃ werden solche Acylreste mit bis zu 10 Kohlenstoffatomen betrachtet.

Die Alkyreste R₅ und R₆, die gegebenenfalls Hydroxygruppen enthalten, sind geradkettige oder verzweigte Alkylreste, insbesondere geradkettige wie zum Beispiel Methyl, Ethyl, Propyl, Butyl, Pentyl, Hexyl, besonders bevorzugt Methyl.

R₇ als C₁₋₅-Alkyl bedeutet geradkettige oder verzweigtkettige, Alkylreste wie sie für R₃ bzw. R₄ bereits genannt wurden. Bevorzugte Alkylreste R₇ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxiden wie Natrium- und Kaliumhydroxid, Erdalkalihydoxide wie Calciumhydoxid, Ammoniak, Amine, wie Ethanolamin, Diethanolamin, Triethanolamin, N-Methylglucamin, Morpholin, Tris-(hydroxymethyl)-methylamin usw.

Um zu den Cyclodextrinclathraten zu gelangen werden die Verbindungen der Formel I mit α-, β- oder γ-Cyclodextrin umgesetzt. Bevorzugt sind β-Cyclodextrinderivate.

Bevorzugte Verbindungen der vorliegenden Erfindung sind Verbindungen der allgemeinen Formel I, wobei dic Reste die folgende Bedeutung haben:
- R₁: ist CH₂OH, CONR₅R₆, COOR₄ mit R₄ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierten Phenylrestes
- A: ist eine trans-CH=CH-CH=CH- oder Tetramethylengruppe;
- B: ist eine geradkettige oder verzweigtkettige gesättigte oder ungesättigte Alkylengruppe mit bis zu 10 C-Atomen, die gegebenenfalls durch Fluor substituiert sein kann, oder die Gruppe 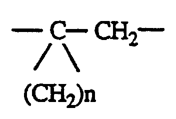 mit n=2-5;
- D: ist eine Direktverbindung, Sauerstoff, Schwefel, eine -C≡C-Gruppe oder eine -CH=CR₇-Gruppe mit R₇ als Wasserstoff, C₁₋₅-Alkyl, Chlor oder Brom;
- B und D: sind gemeinsam eine Direktverbindung;
- R₂: bedeutet Wasserstoff oder einen organischen Säurerest mit 1-15 C-Atomen;
- R₅ und R₆: die oben angegebenen Bedeutungen aufweisen;
- R₃: ist ein Wasserstoffatom, C₁₋₁₀-Alkyl, Cycloalkyl mit 5-6 C-Atomen, ein gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierter Phenylrest und falls
- R₄: ein Wasserstoff bedeutet, deren Salze mit physiologisch verträglichen Basen und Cyclodextrinclathrate.

Besonders bevorzugte Verbindungen der vorliegenden Erfindung sind Verbindungen der allgemeinen Formel I, wobei die Reste folgende Bedeutung haben:
- R₁: ist CH₂OH, CONR₅R₆, COOR₄ mit R₄ in der Bedeutung eines Wasserstoffatoms, eines Alkylrestes mit 1-4 C-Atomen
- R₂: bedeutet Wasserstoff oder einen organischen Säurerest mit 1-6 C-Atomen;
- R₃: ist ein Wasserstoffatom oder C₁₋₁₀-Alkyl;
- R₅ und R₆: die oben angegebenen Bedeutungen aufweisen;
- A: ist eine Trans, trans-CH=CH-CH=CH- oder Tetramethylengruppe;
- B: ist eine geradkettige oder verzweigtkettige Alkylengruppe mit bis zu 5 C-Atomen;
- D: ist eine Direktverbindung oder eine -C≡C-Gruppe oder eine -CH=CR₇-Gruppe mit R₇ als Wasserstoff oder C₁₋₅-Alkyl;
- B und D: sind gemeinsam eine Direktbindung;
und falls R₄ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der allgemeinen Formel I, das dadurch gekennzeichnet ist, daß man einen Alkohol der Formel II 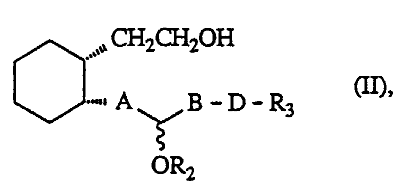 worin A, B, D, R₂ und R₃ die oben angegebene Bedeutung haben, gegebenenfalls nach Schutz freier Hydroxygruppen in R₂ mit einem Alkylhalogenid oder Halogenessigsäurederivat der allgemeinen Formel III,

X-CH₂R₁ (III)

worin X Chlor, Brom oder Iod und R₁ -CH₃, CF₃, COOR₄, CONR₅R₆ oder -CH₂OR₉ darstellt, worin R₉ einen leicht abspaltbaren Etherrest bedeutet, in Gegenwart einer Base verethert und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe verseift und/oder reduziert und/oder eine Carboxylgruppe verestert und/oder eine freie Carboxylgruppe in ein Amid überführt oder eine Carboxylgruppe mit einer physiologisch verträglichen Base in ein Salz überführt.

Als Etherreste R₉ in der Verbindung der Formel II kommen die dem Fachmann geläufigen Reste in Betracht. Bevorzugt sind leicht abspaltbare Etherreste, wie beispielsweise Dimethyl-tert.-butylsilyl, Trimethylsilyl-, Tribenzylsilyl-, Diphenyl-tert.-butylsilyl-, Tetrahydropyranyl-, Tetrahydrofuranyl, und α-Ethoxyethyl, um nur einige zu nennen.

Die Umsetzung der Verbindung der allgemeinen Formel II mit einem Alkylhalogenid oder einem Halogenessigsäurederivat der allgemeinen Formel III wird bei Temperaturen von 0°C bis 100°C, vorzugsweise bei 10°C bis 80°C in einem aprotischen Lösungsmittel oder Lösungsmittelgemisch, beispielsweise Dimethylsulfoxid, Dimethylformamid, Tetrahydrofuran usw. vorgenommen. Als Basen kommen die dem Fachmann für Veretherungen bekannten Basen in Frage, beispielsweise Natriumhydrid, Kalium-tert-butylat, Butyllithium usw..

Die oben genannte Veretherung kann auch vorzugsweise unter PhasentransferBedingungen mit 20-50%iger wäßriger Natriumhydroxid- oder Kaliumhydroxid-Lösung ohne ein zusätzliches Lösungsmittel oder in einem aprotischen Lösungsmittel wie beispielsweise Toluol in Gegenwart eines Phasentransfer-Katalysators wie beispielsweise Tetrabutylammoniumhydrogensulfat bei Temperaturen zwischen 0°C und 90°C, vorzugsweise zwischen 20°C und 60°C durchgeführt werden.

Die Reduktion zu den Verbindungen der Formel I mit R₁ in der Bedeutung einer CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30°C bis zur Siedetemperatur des verwendeten Lösungsmittels, vorzugsweise 0°C bis 30°C vorgenommen.

Die Veresterung der Alkohole der Formel I (R₂=H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise NaH, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, DMSO bei Temperaturen über oder unter Raumtemperatur, zum Beispiel zwischen -80°C bis 100°C, vorzugsweise bei Raumtemperatur, vorgenommen werden.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters, 1979, 399), Jones Reagenz (J. Chem. Soc. 1953, 2555) oder Platin/Sauerstoff (Adv. in Carbohydrate Chem. 17 169 (1962) oder Collins-Oxidation (Tetrahedron Letters 1968, 3363 und anschließende Jones-Oxidation. Die Oxidation mit Pyridiniumdichromat wird bei Temperaturen von 0°C bis 100°C vorzugsweise bei 20°C bis 40°C in einem gegen das Oxidationsmittel inerten Lösungsmittel, beispielsweise Dimethylformamid, durchgeführt.

Die Oxidation mit Jones-Reagenz wird bei Temperaturen von -40°C bis +40°C, vorzugsweise 0°C bis 30°C in Aceton als Lösungsmittel ausgeführt.

Die Oxidation mit Platin/Sauerstoff wird bei Temperaturen von 0°C bis 60°C, vorzugsweise 20°C bis 40°C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden (Asymmetric Synthesis, Vol. 1-5, E.d. J. D. Morrison, Academic Press. Inc. Orlando etc., 1985; Chiral Separarations by HPLC, Ed. A. M. Krstulovic; John Wiley & Sons; New York etc. 1989).

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z. B. Oxalsäure, Essigsäure, Propionsäure u. a., oder in einer wässrigen Lösung einer anorganischen Säure, wie z. B. Salzsäure durchgeführt. Zur Verbesserung der Löslichkeit wird zweckmäßigerweise ein mit Wasser mischbares inertes organisches Lösungsmittel zugesezt. Geeignete organische Lösungsmittel sind z. B. Alkohole, wie Methanol und Ethanol und Ether, wie Dimethoxyethan, Dioxan und Tetrahydrofuran. Tetrahydrofüran wird bevorzugt angewendet. Die Abspaltung wird vorzugsweise bei Temperaturen zwischen 20°C und 80°C durchgeführt. Die Abspaltung der Silyletherschutzgruppen erfolgt beispielsweise mit Tetrabutylammoniumfluorid oder mit Kaliumfluorid in Gegenwart eines Kronenethers (wie zum Beispiel Dibenzo[18]-Krone-6). Als Lösungsmittel sind beispielsweise geeignet Tetrahydrofuran, Diethylether, Dioxan, Dichlormethan, usw. Die Abspaltung wird vorzugsweise bei Temperaturen zwischen 0°C und 80°C durchgeführt

Die Verseifung der Acylgruppen erfolgt beispielsweise mit Alkali- oder Erdalkalicarbonaten oder -hydroxiden in einem Alkohol oder in der wässrigen Lösung eines Alkohols. Als Alkohol kommen niedere aliphatische Alkohole in Betracht, wie z. B. Methanol, Ethanol, Butanol usw., vorzugsweise MethanoL Als Alkalicarbonate und -hydroxide seien Kalium- und Natriumsalze genannt. Bevorzugt sind Kaliumsalze.

Als Erdalkalicarbonate und -hydroxide sind beispielsweise geeignet Calciumcarbonat, Calciumhydroxid und Bariumcarbonat Die Umsetzung erfolgt bei -10°C bis +70°C, vorzugsweise bei +25°C.

Die Einführung der Estergruppe -COOR₄ für R₁, bei welcher R₄ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffs in einem inerten Lösungsmittel, vorzugsweise in Diethylether, mit der 1-carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe -COOR₄ für R₁, bei welcher R₄ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30°C und +50°C, vorzugsweise bei 10°C durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20°C bis +30°C in einer Wasserstoffatmosphäre in Gegenwart eines Edelmetallkatalysators durchgeführt. Als Katalysator ist zum Beispiel 10% Palladium auf Kohle geeignet.

Die Leukotrien-B₄-Derivate der Formel I mit R₄ in der Bedeutung eines Wasserstoffs können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbarcn Lösungsmittels, z, B. Alkohol oder Aceton, das feste anorganische Salz.

Zur Herstellung eines Aminsalzes wird LTB₄-Säure z. B. in einem geeigneten Lösungsmittel, beispielsweise Ethanol, Aceton, Diethylether, Acetonitril oder Benzol gelöst und mindestens die stöchiometrische Menge des Amins der Lösung zugesezt. Dabei fällt das Salz gewöhnlich in fester Form an oder wird nach Verdampfen des Lösungsmittels in üblicher Weise isoliert.

Die Einführung der Amidgruppe -CONHR₅ mit R₅ in der Bedeutung von Alkanoyl erfolgt nach den dem Fachmann bekannten Methoden. Die Carbonsäuren der Formel I (R₄=H), werden zunächst in Gegenwart eines tertiären Amins, wie beispielsweise Triethylamin, mit Chlorameisensäurebutylester in das gemischte Anhydrid überführt. Die Umsetzung des gemischten Anhydrids mit dem Alkalisalz des entsprechenden Amids oder mit Ammoniak (R₅=H) erfolgt in einem inerten Lösungsmittel oder Lösungsmittelgemisch, wie beispielsweise Tetrahydrofuran, Dimethoxyethan, Dimethylformanid, Hexamethylphosphorsäuretriamid, bei Temperaturen zwischen -30°C und +60°C, vorzugsweise bei 0°C bis 30°C. Eine weitere Herstellungsart für die Amide besteht in der Amidolyse des 1-Esters (R₁=-COOR₄) mit dem entsprechenden Amin.

Eine weitere Möglichkeit für die Einführung der Amidgruppe -CONHR₅ besteht in der Umsetzung einer 1-Carbonsäure der Formel I (R₄=H), in der freie Hydroxygrup pen gegebenenfalls intermediär geschützt sind, mit Verbindungen der Formel IV,

O = C = N - R₅ (IV)

worin R₅ die oben angegebene Bedeutung hat.

Die Umsetzung der Verbindung der Formel I (R₄=H) mit einem Isocyanat der Formel IV erfolgt gegebenenfalls unter Zusatz eines tertiären Amins, wie z. B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80°C bis 100°C, vorzugsweise bei 0°C bis 30°C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen im Leukotrien-B₄-Rest, so werden diese OH-Gruppen auch zur Reaktion gebracht. Werden letztlich Endprodukte gewünscht, die freie Hydroxylgruppen enthalten, geht man zweckmäßigerweise von Ausgangsprodukten aus, in denen diese durch vorzugsweise leicht abspaltbare Ether- oder Acylreste intermediär geschützt sind.

Die Trennung von Diastereomeren erfolgt nach den dem Fachmann bekannten Methoden, beispielsweise durch Säulenchromatographie.

Die als Ausgangsmaterial dienenden Verbindungen der Formel II können beispielsweise hergestellt werden, indem man in an sich bekannter Weise cis-1,2-Diacetoxymethyl-cyclohex-4-en oder cis-1,2-Diacetoxymethyl-cyclohexan mit einer Lipase enantioselektiv hydrolysiert (J. B. Jones et aL, J. Chem. Soc. Chem. Commun. 1985, 1563; M. Schneider et al., Tetrahedron Lett. 26, 2073 (1985); H. J. Gais et al., Tetrahedron Lett 28, 3471 (1987)). Das auf diese Weise hergestellte optisch aktive Monoacetat wird anschließend in den Tert.butyldimethylsilylether überführt, gegebenenfalls hydriert and anschließend mit Diisobutylaluminiumhydrid in den Monosilylether der Formel V 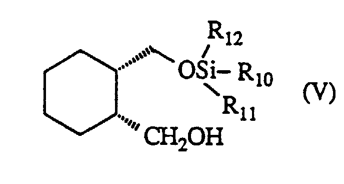 worin R₁₀, R₁₁ und R₁₂ gleich oder verschieden sind und C₁-C₄-Alkyl oder Phenyl bedeuten, überführt.

Durch die Oxidation z. B. mit Collins-Reagenz oder durch das Swern-Verfahren (Tetrahedron Letters 34, 1651 (1978)) wird der Aldehyd der Formel VI erhalten, 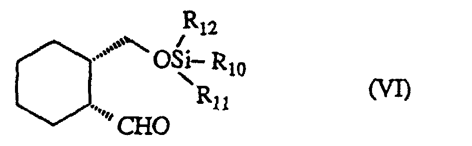 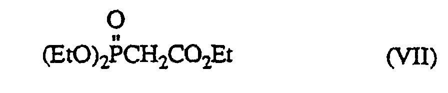 oder 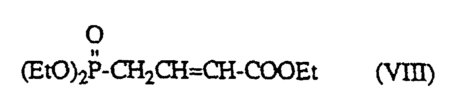 der in einer Wittig-Horner-Olefinierung mit dem Phosphonat der Formel VII und einer Base und gegebenenfalls anschließender Hydrierung sowie anschließender Reduktion der Estergruppe, Oxidation des primären Alkohols, nochmaliger Wittig-Horner-Olefinierung mit dem Phosphonat der Formel VII und gegebenenfalls anschliessender Hydrierung in den Ester der Formcl IX oder einer Wittig-Horner Reaktion des Aldehyds der Formel VI mit einem Phosphonat der Formel VIII überführt wird, wobei A die 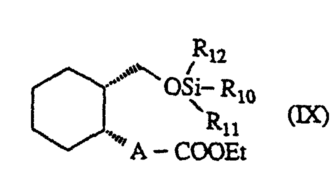 oben angegebene Bedeutung besitzt. Als Basen kommen beispielsweise Kaliumtert.-butylat, Diazabicyclononan, Diazabicycloundecan oder Natriumhydrid in Frage. Reduktion der Estergruppe, beispielsweise mit Diisobutylaluminiumhydrid und anschließende Oxidation des erhaltenen primären Alkohols z. B. mit Braunstein oder Collinsreagenz führt zum Aldehyd der Formel X 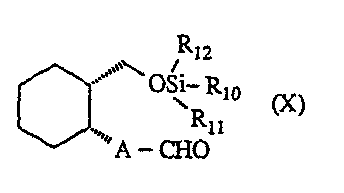 Die metallorganische Umsetzung des Aldehyds der Formel X mit einem Grignardreagenz der Formel XI, worin B, D

X-Mg-B-D-R₃ (XI)

und R₄ die oben angegebenen Bedeutungen aufweisen und X Chlor, Brom oder Iod bedeutet, führt nach Schutz der Hydroxygruppen (beispielsweise durch Acylierung) und gegebenenfalls Diastereomerentrennung zu den Verbindungen der Formel XII 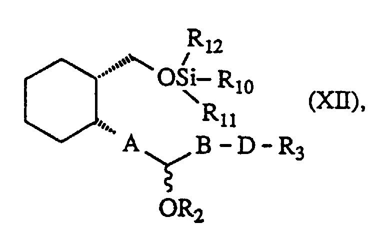 Die Herstellung der für die metallorganische Unsetzung benötigten Verbindung der Formel XI erfolgt durch Reaktion des entsprechenden terminalen Halogenids mit Magnesium. Durch Umsetzung des Silylethers XII mit Tetrabutylammoniumfluorid und gegebenenfalls Diastereomerentrennung wird der Alkohol der Formel XIII erhalten. 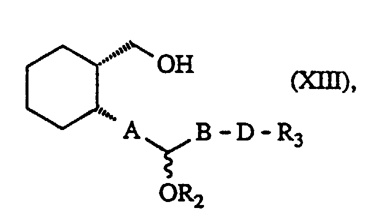 Zu den Verbindungen der Formel XII, worin B eine CH₂-Gruppe und D eine -C≡C-Gruppe oder eine CH=CR₇-Gruppe bedeutet, kann man gelangen, beispielsweise durch eine metallorganische Umsetzung eines Propargylhalogenids und anschließende Alkylierung mit einem entsprechenden Alkylhalogenid und gegebenenfalls anschliessende Lindlar-Hydrierung.

Ein alternativer Aufbau der unteren Kette geht von dem Aldehyd der Formel XIV aus, der aus der Wittig-Horner-Umsetzung des Aldehyds VI und anschließender Reduktion und Oxidation resultierte. 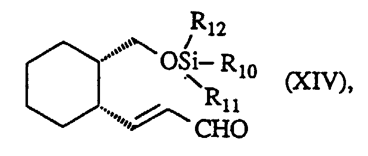 Wittig-Horner-Olefinierung des Aldehyds XIII mit einem Phosphonat der Formel XV 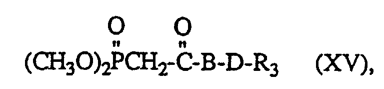 und Reduktion des entstandenen Ketons führte dann zum Alkohol der Formel XII und nach Acelierung und Silyletherspaltung zum Alkohol der Formel III, der gegebenenfalls in die Diastereomeren getrennt werden kann.

Die Verbindungen der allgemeinen Formel XIII sind in der DE-A 42 27 790.6 beschrieben oder können gemäß des in DE-A 42 27 790.6 vorgestellten Verfahrens hergestellt werden.

Nach Tosylierung des Alkohols der allgemeinen Formel XIII und Umsetzung mit Cyanid in Dimethylsulfoxid erhält man das Nitril der Formel XVI, welches gegebenenfalls durch Verseifung des Säurerestes (OR₂) und anschließende Silylierung der freien Hydroxygruppe geschützt werden kann. 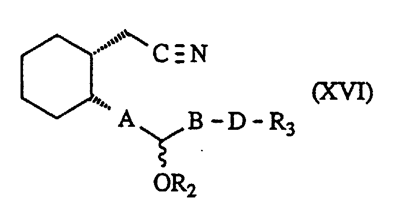 Durch Reduktion der Nitrilgruppe in der Verbindung der Formel XVI, beispielsweise mit Diisobutylaluminiumhydrid und Natriumborhydrid wird dann der Alkohol der allgemeinen Formel II erhalten.

Der Einbau der chemisch und metabolisch labilen cis-Δ^{6,7}-Doppelbindung des LTB₄ in einen cis-1,2-substituierten Cyclohexylring führt zu einer Stabilisierung, wobei insbesondere durch weitere Derivatisierung der funktionellen Gruppen und/oder strukturelle Veränderungen der unteren Seitenkette, LTB₄-Derivate erhalten wurden, die als LTB₄-Antagonisten wirken können (DE-A 39 17 597 und DE-A 42 27 790.6).

Es wurde nun gefunden, daß durch Substitution der Methylengruppe in der 3- Position (Zählweise beginnend beim Caboxyl-C-Atom mit 1) und Weglassen der Hydroxygruppe in der 5-Position in derartigen Leukotrien B₄-Derivaten eine längere Wirkungsdauer, größerre Selektivität und bessere Wirksamkeit erzielt werden kann.

Die Verbindungen der Formel I wirken antientzündlich, antiallergetisch und antiproliferativ. Daneben besitzen sie antimykotische Eigenschaften. Folglich stellen die neuen Leukotrien-B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien-B₄-Derivate der Formel I eignen sich in Kombination mit den in der galenischen Pharmazie üblichen Hilfs- und Trägermitteln zur lokalen Behandlung von Erkrankungen der Haut, bei denen Leukotriene eine wichtige Rolle spielen, z. B.: Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen. Außerdem sind die neuen Leukotrien-B₄-Antagonisten zur Behandlung der multiplen Sklerose und der Symptome des Schocks geeignet.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Salben, Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0,0001% bis 3% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise 0,1 bis 100 mg Wirkstoff enthalten oder oral appliziert werden oder in Form von Suspensionen, die vorzugseise 1-200 mg Wirkstoff pro Dosiseinheit enthalten und rektal appliziert werden auch zur Behandlung von Erkrankungen innerer Organe, bei denen Leukotriene eine wichtige Rolle spielen, wie z. B.: allergische Erkrankungen des Darmtraktes, wie der Colitis ulcerosa und der Colitis granulomatosa.

In diesen neuen Applikationformen eignen sich die neuen LTB₄-Derivate neben der Behandlung von Erkrankungen innerer Organe mit entzündlichen Prozessen auch zur Behandlung von Erkrankungen bei denen leukotrienabhängig das gesteigerte Wachstum und die Neubildung von Zellen im Vordergrund stehen. Beispiele sind Leukämie (gesteigertes Wachstum weißer Blutkörperchen) oder Artherosklerose (gesteigertes Wachstum glatter Muskelzellen von Blutgefäßen).

Die neuen Leukotrien-B₄-Derivate können auch in Kombination, wie z. B. mit Lipoxygenasehemmern, Cyclooxygenasehemmern, Glukokortikoiden, Prostacyclinagonisten, Thromboxanantagonisten, Leukotrien D₄-Antagonisten, Leukotrien E₄-Antagonisten, Leukotrien-F₄-Antagonisten, Phosphodiesterasehemmern, Calciumantagonisten, PAF-Antagonisten oder anderen bekannten Therapieformen der jeweiligen Erkrankungen, verwendet werden.

Die nachfolgenden Ausführungsbeispiele dienen zur näheren Erläuterung des erfindungsgemäßen Verfahrens. In den Beispielen wurden nicht näher charakterisierte Diastereoisomere in der 12-Position als polar beziehungsweise unpolar charakterisiert (z. B. Diastereomer unpol (12)).

### Beispiel 1

### 3-Oxa-5-[cis-(2R)-2-((1E,3E)-(5R)-5-hydroxy-5-cyclohexyl-1,3-pentadienyl)-1R)-cyclohexyl]-pentansäure-tert.-butylester Diastereomer pol (12)

Zu einer Lösung von 420 mg (5R)-5-Tert.-butyl-dimethylsilyloxy-1-[cis-(1R)-1-(2-hydroxyethyl)-(2R)-cyclohex-2-yl]-5-cyclohexyl-(1E,3E)-pentadien (Diastereomer pol (12)) in 7,1 ml Toluol fügt man 1000 mg Bromessigsäure-tert.-butylester, 15,3 mg Tetrabutylammoniumhydrogensulfat und 3,1 ml einer 25%igen wässrigen Natronlauge und rührt 24 Stunden bei Raumtemperatur unter Argon. Anschließend wird mit Diethylether verdünnt und mit Wasser neutralgewaschen. Man trocknet über Natriumsulfat, dampft im Vakuum ein und reinigt den Rückstand durch Säulenchromatographie an Kieselgel. Mit Hexan/Ether (9+1) erhält man 490 mg des Tert.-butylesters als farbloses Öl.
IR (CHCl₃): 2912, 2850, 1740, 1378, 990, 835 cm⁻¹.

Zur Silyletherspaltung fügt man zu einer Lösung von 490 mg des vorstehend hergestellten Silylethers in 23 ml Tetrahydrofuran 897 mg Tetrabutylammoniumfluorid und rührt 4 Stunden bei 24°C unter Argon. Man fügt nochmal 1,4 g Tetrabutylammoniumfluorid zu und rührt 20 Stunden bei 24°C. Anschließend verdünnt man mit 200 ml Diethylether, wäscht dreimal mit Sole, trocknet über Matriumsulfat und dampft im Vakuum ein. Den Rückstand reinigt man durch Säulenchromatographie an Kieselgel. Mit Hexan/Essigsäureethylester (9+1) erhät man 310 mg der Titelverbindung als farbloses Öl.
IR: 3610, 2930, 2858, 1745, 1450, 990, 945 cm⁻¹.

Das Ausgangsmaterial für die obige Titelverbindung wird wie folgt hergestellt:
1a)

### (5R)-5-Tert.-butyl-dimethylsilyloxy-1-[cis-(1R)-1-(2-hydroxyethyl)-(2R)-cyclohex-2-yl]-5-cyclohexyl-(1E,3E)-pentadien Diastereomer pol (12)

Zu einer Lösung von 3,7 g (5R)-5-Acetoxy-1-[cis-(1S)-hydroxymethyl-(2S)-cyclohex-2-y 1]-5-cyclohexyl-(1E,3E)-pentadien (Diastereomer pol(12)) (diese Verbindung ist in der DE-A 42 27 790.6 beschrieben oder kann gemäß DE-A 42 27 790.6 hergestellt werden; Diastereomer pol (12) bedeutet, daß nach der chromatographischen Diastereomerentrennung dem polaren allylischen Alkohol in der 12-Position (Leukotrien B₄-Zählweise) die 12β (hier 12R)-Konfiguration zugeordnet wurde) in 19,6 ml Pyridin fügt man bei 0°C 4,4 g p-Toluolsulfonsäurechlorid und rührt 24 Stunden bei 24°C. Anschließend fügt man zwei Stückchen Eis dazu, rührt 2 Stunden und verdünnt mit 400 ml Diethylether. Man schüttelt nacheinander mit 20 ml Wasser, zweimal mit je 20 ml 10%iger wässriger Schwefelsäure, einmal mit 20 ml Wasser, einmal mit 20 ml 5%iger wässriger Natiumbicarbonatlösung und dreimal mit je 20 ml Wasser. Man trocknet mit Natriumsulfat und dampft im Vakuum ein. Dabei erhält man 5,4 g des Tosylats, welches ohne weitere Reinigung weiter verwendet wird.

Zur Nitrileinführung löst man 5,4 g des vorstehend hergestellten Tosylats in 46 ml Dimethylsulfoxid, fügt 1,2 g Natriumcyanid zu und rührt 24 Stunden bei 85°C unter Argon. Anschließend kühlt man ab, gießt auf 50 ml Eiswasser, extrahiert dreimal mit je 200 ml Essigsäureethylester, wäscht die organische Phase mit Wasser neutral, trocknet mit Natriumsulfat und dampft im Vakuum ein. Den Rückstand reinigt man durch Säulenchromatographie an Kieselgel. Dabei erhält man 3,24 g des Nitrils als farbloses Öl.
IR: 2923, 2850, 2245, 1735, 1240, 995, 975 cm⁻¹.

Zur Acetatabspaltung fügt man zu einer Lösung von 1,5 g des vorstehend hergestellten Nitrils in 66 ml Methanol 1,3 g wasserfreies Natriumcarbonat und rührt 4 Stunden bei 24°C unter Argon. Anschließend säuert man mit 10%iger wässriger Schwefelsäure auf pH 6 an, engt im Vakuum ein und nimmt den Rückstand in Diethylether auf. Man wäscht mit Sole neutral, trocknet über Natriumsulfat und engt im Vakuum ein. Dabei erhält man 1,5 g des Alkohols, welcher ohne weitere Reinigung verwendet wird.

Zur Silyetherbildung fügt man zu einer Lösung von 1,5 g des vorstehend hergestellten Alkohols in 12,6 ml Dimethylformamid 1 g Imidazol und 1,14 g tert.-Butyldimethylsilylchlorid und rührt 24 Stunden bei 24°C. Man verdünnt mit Diethylether, schüttelt mit 20 ml einer 10%igen wässrigen Schwefelsäure, mit 20 ml Wasser, mit 20 ml einer gesättigten wässrigen Natriumbicarbonatlösung und wäscht mit Wasser neutral. Man trocknet über Natriumsulfat und dampft im Vakuum ein. Den Rückstand reinigt man durch Säulenchromatographie an Kieselgel. Dabei erhält man 1,83 g des Silyethers als farbloses Öl.
IR: 2930, 2858, 2250, 993, 838 cm⁻¹.

Zur Reduktion der Nitrilgruppe tropft man bei -70°C zu einer Lösung von 1,83 g des vorstehend hergestellten Silyethers in 48 ml Toluol 15 ml einer ca. 1,2 molaren Lösung von Diisobutylaluminiumhydrid in Toluol und rührt 30 Minuten bei -70°C. Anschließend tropft man 1,5 ml Isopropanol und dann 7,5 ml Wasser zu, rührt 2 Stunden bei 22°C, filtriert, wäscht mit Toluol und dampft im Vakuum ein. Der so erhaltene Aldehyd wird in 45 ml Methanol gelöst, bei -20°C mit 1,05 g Natriumborhydrid versetzt und 50 Minuten bei -20°C gerührt. Anschließend verdünnt man mit Diethylether, wäscht mit Wasser neutral, trocknet über Natriumsulfat und dampft im Vakuum ein. Den Rückstand reinigt man durch Säulenchromatographie an Kieselgel. Dabei erhält man 800 mg der Titelverbindung als farbloses Öl.
IR: 3350, 2923, 2853,990,835 cm⁻¹.

### Beispiel 2

### 3-Oxa-5-[cis-(2R)-2-((1E,3E)-(5R)-5-hydroxy-5-cyclohexyl-1,3-pentadienyl)-(1R)-cyclohexyl]-pentansäure Diastereomer pol(12)

Zu einer Lösung von 310 mg des nach Beispiel 1 hergestellten Tert.-butylesters in 7,6 ml Methanol fügt man bei 24°C 7,6 ml einer 0,5 normalen Natronlauge und rührt 24 Stunden bei 24°C. Anschließend säuert man mit 10%iger wässriger Schwefelsäure auf pH 5-6 an und verdünnt mit Essigsäureethylester. Man wäscht die organische Phase mit Sole neutral, trocknet über Magnesiumsulfat und engt im Vakuum ein. Den Rückstand reinigt man durch Säulenchromatographie an Kieselgel. Dabei erhält man 229 mg der Titelverbindung als farbloses Öl.
IR: 3450, 2929, 2857, 1738, 1450, 1138, 990, 950 cm⁻¹.

### Beipiel 3

### 3-Oxa-5-[cis-(2R)-2-((1E,3E)-(5S)-5-hydroxy-5-cyclohexyl-1,3-pentadienyl)-(1R)-cyclohexyl]-pentansäure-tert.-butylester Diastereomer unpol(12)

In Analogie zu dem in Beispiel 1 beschriebenen Verfahren erhält man aus 335 mg (5S)-5-Tert.-butyl-dimethylsilyloxy-1-[cis-(1R)-1-(2-hydroxyethyl)-(2R)-cyclohex-2-yl]-5-cyclohexyl-(1E,3E)-pentadien (Diastereomer unpol 12) 220 mg der Titelverbindung als farbloses Öl.
IR: 3660, 2928, 2858, 1745, 1450, 991, 946 cm⁻¹.

Das Ausgangsmaterial für die obige Titelverbindung wird wie folgt hergestellt:
3a)

### (5S)-5-Tert.-butyl-dimethylsilyloxy-1-[cis-(1R)-1-(2-hydroxyethyl)-(2R)-cyclohex-2-yl]-5-cyclohexyl-(1E,3E)-pentadien Diastercomer unpol(12)

In Analogie zu Beispiel 1a erhält man aus 3,3 g (5S)-5-Acetoxy-1-[cis-(1S)-1-hy-droxymethyl-(2S)-cyclohex-2-yl]-5-cyclohexyl-(1E,3E)-pentadien (Diastereomer unpol(12) (diese Verbindung ist in der DE-A 42 27 790.6 beschrieben oder kann gemäß DE-A 42 27 790.6 hergestellt werden. Diastereomer unpol (12) bedeutet, daß nach der chromatographischen Diastereomerentrennung dem unpolaren allylischen Alkohol in der 12-Position (Leukotrien B₄-Zählweise) die 12α (hier 12S)-Konfiguration zugeordnet wurde) 718 mg der Titelverbindung als farbloses Öl.
IR: 3445, 2945, 2855, 990, 835 cm⁻¹.

### Beispiel 4

### 3-Oxa-5-[cis-(2R)-2-((1E,3E)-(5S)-5-hydroxy-5-cyclohexyl-1,3-pentadienyl)-(1R)-cyclohexyl]-pentansäure Diastereomer unpol(12)

In Analogie zu Beispiel 2 erhält man aus 210 mg des nach Beispiel 3 hergestellten tert.-Butylesters 125 mg der Titelverbindung als farbloses Öl.
IR: 3450, 2930, 2858, 1738, 1450, 992, 950 cm⁻¹.

### Beispiel 5

### 3-Oxa-5-[cis-(2R)-2-((1E,3E)-(5S)-5-hydroxy-6,6-trimethylen-9-phenyl-1,3-nonadien-8-inyl)-(1R)-cyclohexyl]-pentansäure-tert.-butylester Diastereomer pol(12)

Zu einer Lösung von 4 g (5S)-5-Tert.-butyldimethylsi1yloxy-1-[cis-(1R)-1-(2-hydroxyethyl)-(2R)-cyclohex-2-yl]-6,6-trimethylen-9-phenyl-(1E,3E)-1,3-nonadien-8-in (Diastereomer pol (12)) in 50 ml Toluol fügt man 7,9 g Bromessigsäure-tert.butylester, 121 mg Tetrabutylammoniumhydrogensulfat und 25 ml einer 25%igen wässrigen Natronlauge und rührt 24 Stunden bei Raumtemperatur unter Argon. Anschliessend wird mit Diethylether verdünnt und mit Wasser neutral gewaschen. Man trocknet über Magnesiumsulfat, dampft im Vakuum ein und reinigt den Rückstand durch Säulenchromatographie an Kieselgel. Mit Hexan/Ether (9+1) erhält man 3,9 g des Tert.-butylcstcrs als farbloses Öl.
IR: 2925, 2850, 1740, 1378, 990, 835 cm⁻¹.

Zur Silyetherspaltung fügt man zu einer Lösung von 3,9 g des vorstehend hergestellten Silylethers in 200 ml Tetrahydrofuran 16,1 g Tetrabutylammoniumfluorid und rührt 4 Stunden bei 24°C. Anschließend verdünnt man mit Diethylether und wäscht dreimal mit Sole. Man trocknet über Natriumsulfat und dampft im Vakuum ein. Den Rückstand reinigt man durch Säulenchromatographie an Kieselgel. Mit Hexan/Essigsäureethylester (8+2) erhält man 2,7 g der Titelverbindung als feste Substanz (Schmelzpunkt 53°C).
IR: 3610, 2930, 2860, 1745, 1600, 1370, 990 cm⁻¹.

Das Ausgangsmaterial für die obige Titelverbindung wird wie folgt hergesellt:
5a)

### (5S)-5-Tert.-butyldimethylsilyloxy-1-[cis-(1R)-1-(2-hydroxyethyl)-(2R)-cyclohex-2-yl]-6,6-trimethylen-9-phenyl-(1E,3E)-1,3-nonadien-8-in Diastereomer pol (12)

In Analogie zu Beispiel 1a erhält man aus 4,98 g (5S)-5-Acetoxy-1-[cis-(1S)-1-hydroxymethyl-)-(2S)-cyclohex-2-yl]-6,6-trimethylen-9-phenyl-(1E,3E)-1,3-nonadien-8-in (Diastereomer pol (12)) (dicse Verbindung ist in der DE-A 42 27 790.6 beschrieben oder kann gemäß DE-A 42 27 790.6 hergestellt werden) 4 g der Titelverbindung als farbloses Öl.
IR: 3620, 2922, 2850, 1600, 990, 835 cm⁻¹.

### Beispiel 6

### 3-Oxa-5-[cis-(2R)-2-((1E,3E)-(5S)-5-hydroxy-6,6-trimethylen-9-phenyl-1,3-nonadien-8-inyl)-(1R)-cyclohexyl]-pentansäure Diastereomer pol(12)

In Analogie zu Beispiel 2 erhält man aus 1,68 g des nach Beispiel 5 hergestellten tert.-Butylesters 1,36 g der Titelverbindung als farbloses Öl.
IR: 3610, 3520, 1730, 1600, 990 cm⁻¹.

### Beipiel 7

### 3-Oxa-5-[cis-(2R)-2-((1E,3E)-(5S)-5-hydroxy-6,6-trimethylen-9-phenyl-1,3-nonadien-8-inyl)-(1R)-cyclohexyl]-pentansäure-tert.-butylester Diastereomer unpol(12)

In Analogie zu dem in Beispiel 1 beschriebenen Verfahren erhält man aus 2,25 g (5R)-5-tert.-butyl-dimethylsilyoxy-1-[cis-(1R)-1-(2-hydroxyethyl)-(2R)cyclohex-2-yl ]-6,6-trimethylen-9-phenyl-(1E,3E)-1,3-nonadien-8-in (Diastereomer unpol (12)) 1,36 g der Titelverbindung als farbloses Öl.
IR: 3600, 2930, 2859, 1745, 1600, 990 cm⁻¹.

Das Ausgangsmaterial für die obige Titelverbindung wird wie folgt hergestellt:
7a)

### (5S)-5-Tert.-butyl-dimethylsilyloxy-1-[cis-(1R)-(2-hydroxyethyl)-(2R)-cyclohex2-yl]-6,6-trimethylen-9-phenyl-(1E,3E)-1,3-nonadien-8-in Diasteromer unpol(12)

In Analogie zu Beispiel 1a erhält man aus 3,48 g (5R)-5-Acetoxy-1-[cis-(1S)-1-hydroxymethyl-(2S)-cyclohex-2-yl]-6,6-trimethylen-9-phenyl-(1E,3E)-1,3-nonadien-8-in (Diastereomer unpol (12)) (diese Verbindung ist in der DE-A 42 27 790.6 beschrieben) 2,27 g der Titelverbindung als Öl.
IR: 3620, 2923, 2850, 1598, 990, 835 cm⁻¹.

### Beipiel 8

### 3-Oxa-5-[cis-(2R)-2-((1E,3E)-(5R)-5-hydroxy-6,6-trimethylen-9-phenyl-1,3-nonadien-8-inyl)-(1R)-cyclohexyl]-pentansäure Diastereomer unpol(12)

In Analogie zu Beispiel 2 erhält man aus 1 g des nach Beispiel 7 hergestellten tert.-Butylesters 790 mg der titelverbindung als farbloses Öl.
IR: 3620, 3500, 2924, 2853, 1730, 1600, 990 cm⁻¹.

### Beispiel 9

### 3-Oxa-5-[cis-(2R)-2-((1E,3E)-(5R,8S)-5-hydroxy-8,12-dimethyl-1,3,1,1-tridecatrienyl-(1R)-cyclohexy]-pentansäure-tert.-butylester Diastereomer pol(12)

In Analogie zu dem in Beispiel 1 beschriebenen Verfahren erhält man aus 720 mg (5R)-5-Tert.butyl-dimethylsilyloxy-1-[cis-(1R)-1-(2-hydroxyethyl)-(2R)-cyclohex-2-yl]-8,12-dimethyl-(1E,3E,11E)-tridecadien (Diastereomer pol (12)) 280 mg der Titelverbindung als farbloses Öl.
IR (Film): 3500, 2929, 2860, 1742, 1599, 1369, 991 cm⁻¹.

Das Ausgangsmaterial für die obige Titelverbindung wird wie folgt hergestellt:
9a)

### (5R,8S)-5-Tert.-butyl-dimethylsilyoxy-1-[cis-((1R)-1-(2-hydroxyethyl)-(2R)-cyclohex-2-yl]-8,12-dimethyl-(1E,3E,11E)-tridecatrien (Diastereomer pol (12))

In Analogie zu Beispiel 1a erhält man aus 1,95 g (SR,8S)-5-Acetoxy-1-[cis-(1S)-1-hydroxymethyl-(2S)-cyclohex-2-yl]-8,12-dimethyl-(1E,3E,11E)-tridecatrien (Diastereomer pol (12)) (diese Verbindung ist in der DE-A 42 27 790.6 beschrieben oder kann gemäß DE-A 42 27 790.6 hergestellt werden) 732 mg der Titelverbindung als farbloses Öl.
IR: 3440, 2948, 2856, 990, 835 cm⁻¹.

### Beispiel 10

### 3-Oxa-5-[cis-(2R)-2-((1E,3E)-(5R,8S)-5-hydroxy-8,12-dimethyl-1,3,11-tridecatrienyl-(1R)-cyclohexyl]-pentansäure Diastereomer pol(12)

In Analogie zu Beispiel 2 erhält man aus 0,55 g des nach Beispiel 9 hergestellten tert.-Butylesters 0,28 g der Titelverbindung als farbloses Öl.
IR: 3450, 2923, 2855, 1733, 990 cm⁻¹.

### Beispiel 11

### 3-Oxa-5-[cis-(2R)-2-((1E,3E)-(5RS,7RS)-5-hydroxy-7-methyl-1,3-tridecadien-9-inyl)-(1R)-cyclohexyl]-pentansäure-tert.-butylester Diastereomer unpol(12)

In Analogie zu dem in Beispiel 1 beschriebenen Verfahren erhält man aus 1,26 g (5RS)-5-Tert.-butyl-dimethylsilyloxy-1-[cis-(1R)-1-(2-hydroxyethyl)-(2R)-cyclohex-2-yl]-7-methyl-1,3-tridecadien-9-in (Diastereomer unpolar A) 970 mg der Titelverbindung als farbloses Öl .
IR: 3450, 2923, 2860, 1748, 1368, 1135, 990, 943 cm⁻¹.

Das Ausgangsmaterial für die obige Titelverbindung wird wie folgt hergestellt:
11a)

### (5RS,7RS)-5-Tert.-butyl-dimethylsilyloxy-1-[cis-(1R)-1-(2-hydroxyethyl)-(2R)-cyclohex-2-yl]-7-methyl-1,3-tridecadien-9-in Diastereomer unpol A

In Analogie zu Beispiel 1a erhält man aus 2,28 g (5RS,7RS)-Acetoxy-1-[cis-(1S)-1-hydroxymethyl-(2S)-cyclohex-2-yl]-7-methyl-1,3-tridecadien-9-in (Diastereomer unpol A) (diese Verbindung ist in der DE-A 42 27 790.6 beschrieben oder kann gemäß DE-A 42 27 790.6 hergestellt werden) 1,26 g der Titelverbindung als farbloses Öl.
IR: 3540, 2930, 2860, 990, 838, 777 cm⁻¹.

### Beispiel 12

### 3-Oxa-5-[cis-(2R)-2-((1E,3E)-(5RS,7RS)-5-hydroxy-7-methyl-1,3-tridecadien-9-inyl)-(1R)-cyclohexyl]-pentansäure Diastercomer unpol A

In Analogie zu Beispiel 2 erhält man aus 770 mg des nach Beispiel 11 hergestellten tert.-Butylesters 643 mg der Titelverbindung als farbloses Öl.
IR: 3440. 2930, 2860, 1735, 1135, 990 cm⁻¹

### Beispiel 13

### 3-Oxa-5-[cis-(2R)-2-((1E,3E)-(5R,6RS)-5-hydroxy-6-methyl-9-phenyl-1,3-nonadien-8-inyl)-(1R)-cyclohexyl]-pentansäure-tert.-butylester Diastereomer pol(12)

In Analogie zu Beispiel 1 erhält man aus 2,9 g (5R,6RS)-5-Acetoxy-1-[cis-(1S)-1-hydroxymethyl)-(2S)-cyclohex-2-yl]-6-methyl-9-phenyl-(1E,3E)-1,3-nonadien-8-in (Diastereomer pol (12)) (diese Verbindung ist in der DE-A 42 27 790.6 beschrieben oder kann gemäß DE-A 42 27 790.6 hergestellt werden) 1,1 g der Titelverbindung als farbloses Öl.
IR: 3600, 2930, 2858, 1746, 1600, 1371, 990 cm⁻¹.

### Beispiel 14

### 3-Oxa-5-[cis-(2R)-2-((1E,3E)-(5R,6RS)-5-hydroxy-6-methyl-9-phenyl-1,3-nonadien-8-inyl)-(1R)-cyclohexyl]-pentansäure Diastereomer pol(12)

In Analogie zu Beispiel 2 erhält man aus 0,6 g des nach Beispiel 13 hergestellten tert.-Butylesters 0,45 g der Titelverbindung als farbloses Öl.
IR: 3600, 3510, 1731, 1600, 990 cm⁻¹.

### Beispiel 15

### 3-Oxa-5-[cis-(2R)-2-((1E,3E)-(5R)-5-hydroxy-5(cyclohexyl-1,3-pentadienyl)-(1R)-cyclohexyl]-pentansäure-methylester Diastereomer pol(12)

Zu einer Lösung von 360 mg der nach Beispiel 2 hergestellten Säure in 10 ml Diethylether tropft man bei 0°C eine etherische Diazomethanlösung bis zur bleibenden Gelbfärbung und rührt 10 Minuten bei 0°C. Anschließend dampft man im Vakuum ein und reinigt den Rückstand durch Säulenchromatographie an Kieselgel. Mit Hexan/Essigsäureethylester (4+1) erhält man 360 mg der Titelverbindung als farbloses Öl.
IR: 3450, 2922, 2850, 1755, 1450, 1260, 1140, 990 cm⁻¹.

### Beispiel 16

### 3-oxa-5-[cis-(2R)-2-((1E,3E)-(5S)-5-hydroxy-6,6-trimethylen-9-phenyl-1,3-nonadien-8-inyl)-(1R)-cyclohexyl]-pentansäuremethylester Diastereomer pol(12)

In Analogie zu Beispiel 15 erhält man aus 100 mg der nach Beispiel 6 hergestellten Carbonsäure 94 mg der Titelverbindung als farbloses Öl.
IR: 3500, 2922, 2850, 2215, 1755, 1598, 1210, 991 cm⁻¹.

### Beispiel 17

### 3-Oxa-5-[cis-(2R)-2-((1E,3E)-(5S)-5-hydroxy-6,6-trimethylen-9-phenyl-1,3-nonadien-8-inyl)-(1R)-cyclohexyl]-pentansäurephenacylester Diastereomer pol(12)

Zu einer Lösung von 118 mg der nach Beispiel 6 hergestellten Carbonsäure in 2 ml Aceton fügt man 63 mg Phenacylbromid und 1 ml einer Lösung von Triethylamin in Aceton (Herstellung: Man löst 350 mg Triethylamin in 10 ml Aceton). Man rührt 4 Stunden bei 24°C und verdünnt anschließend mit 50 ml Diethylether. Man schüttelt zweimal mit Wasser, trocknet über Magnesiumsulfat und dampft im Vakuum ein. Den Rückstand reinigt man durch Chromatographie an Kieselgel. Mit Hexan/Essigsäureethylester (3+2) erhält man 144 mg der Titelverbindung als farbloses Öl. IR: 3510, 2923, 2852, 2220, 1767, 1705, 1598, 990, 967 cm⁻¹.

### Beispiel 18

### 3-Oxa-5-[cis-(2R)-2-((1E,3E)-(5R)-5-hydroxy-5-cyclohexyl-1,3-pentadienyl)-(1R)-cyclohexyl]-pentansäure-(2,3-dihydroxy-propyl)-amid Diastereomer pol(12)

Zu einer Lösung von 150 mg des nach Beispiel 6 hergestellten Methylesters in 3 ml Acetonitril fügt man 450 mg 3-Amino-propan-1,2-diol und rührt 24 Stunden bei 80°C. Anschließend wird im Vakuum eingedampft und der Rückstand durch Säulenchromatographie an Kieselgel gereinigt Mit Dichlormethan/Methanol (9+1) erhält man 165 mg der Titelverbindung als farbloses Öl.
IR: 3400, 2922, 2852, 1660, 1543, 990 cm⁻¹.

### Beispiel 19

### 3-Oxa-5-[cis-(2R)-2-((1E,3E)-(5R)-5-hydroxy-5-cyclohexyl-1,3-pentadienyl)-(1R)-cyclohexyl]-pentansäure-[tris-(hydroxymethyl)-methyl]-amid Diastereomer pol(12)

Zu einer Lösung von 180 mg des nach Beispiel 6 hergestellten Methylesters in 0,1 ml Dimethylsulfoxid fügt man 390 mg Trishydroxymethylaminomethan und rührt 24 Stunden bei 24°C. Anschließend wird die Reaktionsmischung durch Säulenchromatographie an Kieselgel gereinigt. Mit Dichlormethan/Methanol (9+1) erhält man 217 mg der Titelverbindung als farbloses Öl.
IR: 3350, 2922, 2850, 1655, 1530, 990 cm⁻¹.

### Beispiel 20

### 3-Oxa-5-[cis-(2R)-2-((1E,3E)-(5R)-5-hydroxy-5-cyclohexyl-1,3-pentadienyl)-(1R)-cyclohexyl]-pentansäure-butylamid Diastereomer pol(12)

100 mg des nach Beispiel 1 hergestellten tert.-Butylesters kocht man 48 Stunden unter Argon in 2 ml Butylamin am Rückfluß. Anschließend dampft man im Vakuum ein und reinigt den Rückstand durch Säulenchromatographie. Mit Hexan/Essigsäureethylester (1+1) erhält man 63 mg der Titelverbindung als farbloses Öl.
IR: 3600, 3420, 2925, 2858, 1665, 1533,1448, 990 cm⁻¹.

### Beispiel 21

### Tris-(hydroxymethyl)-aminomethansalz von 3-Oxa-5-[cis-(2R)-2-((1E,3E)-(5R)-5-hydroxy-5-cyclohexyl-1,3-pentadienyl)-(1R)-cyclohexyl]-pentansäure Diastereomer pol(12)

Zu einer Lösung von 100 mg der nach Beispiel 2 hergestellten Carbonsäure in 17,3 ml Acetonitril fügt man bei 80°C 0,054 ml einer wässrigen Tris-(hydroxymethyl)-amino-methan-Lösung (Herstellung: Man löst 8,225 g Tris-(hydroxymethyl)-aminomethan in 15 ml Wasser), rührt 1 Stunde bei 80°C, 1 Stunde bei 55°C, 48 Stunden bei 45°C und 48 Stunden bei 24°C. Man saugt die gebildeten Kristalle ab, wäscht mit etwas Acetonitril und trocknet die Kristalle bei 24°C im Vakuum. Dabei erhät man 134 mg der Titelverbindung als farblose Kristalle (Schmeltpunkt:113-114°C).
IR: 3350, 2922, 2850, 1590 (breit), 1450, 992 cm⁻¹.

### Beispiel 22

### Tris-(hydroxymethyl)-aminomethansalz von 3-Oxa-5-[cis-(2R)-2-((1E,3E)-(5R)-5-hydroxy-6,6-trimethylen-9-phenyl-1,3-nonadien-8-inyl-(1R)-cyclohexyl]-pentansäure Diastereomer pol(12)

In Analogie zu Beispiel 21 erhält man aus 124 mg der nach Beispiel 6 hergestellten Carbonsäure 149 mg der Titelverbindung als farblose Kristalle (Schmelzpunkt 114-116°C).
IR (KBr): 3350, 2922, 2850, 1588 (breit), 992 cm⁻¹.

## Patentansprüche

1. Leukotrien-B₄-Derivate der allgemeinen Formel I, 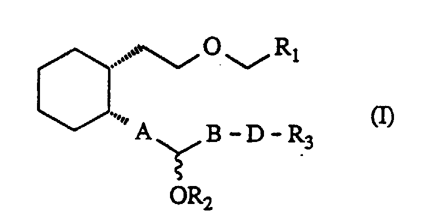 worin
R₁ CH₂OH, CH₃, CF₃, COOR₄, CONR₅R₆,
R₂ H oder ein organischer Säurerest mit 1-15 C-Atomen darstellt,
R₃ H, gegebenenfalls einfach oder mehrfach substituiertes C₁-C₁₄-Alkyl, C₃-C₁₀-Cycloalkyl, gegebenenfalls unabhängig voneinander einfach oder mehrfach durch Halogen, Phenyl, C₁-C₄-Alkyl, C₁-C₄-Alkoxy, Fluormethyl, Chlormethyl, Trifluormethyl, Carbonyl, Carboxyl oder Hydroxy substituierter C₆-C₁₀-Arylrest oder ein 5-6 gliedriger aromatischer heterocyclischer Ring mit wenigstens 1 Heteroatom symbolisieren,
R₄ Wasserstoff, C₁-C₁₀ Alkyl, C₃-C₁₀ Cycloalkyl, gegebenenfalls durch 1-3 Halogen, Phenyl, C₁-C₄-Alkyl, C₁-C₄-Alkoxy, Fluormethyl, Chlormethyl, Trifluormethyl, Carboxyl oder Hydroxy substituierter C₆-C₁₀-Arylrest, CH₂-CO-(C₆-C₁₀) Aryl oder ein 5-6 gliedriger Ring mit wenigstens 1 Heteroatom bedeutet,
A eine trans, trans-CH=CH-CH=CH, eine -CH₂CH₂-CH=CH- oder eine Tetramethylengruppe,
B eine C₁-C₁₀- gerad- oder verzweigtkettige Alkylengruppe, die gegebenenfalls durch Fluor substituiert sein kann oder die Gruppe 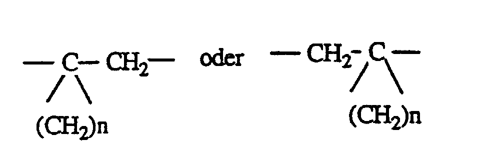 symbolisiert,
D eine Direktbindung, Sauerstoff, Schwefel, -C≡C-, -CH=CR₇, oder gemeinsam mit
B auch eine Direktbindung bedeuten können,
R₅ und R₆ gleich oder verschieden sind und H oder C₁-C₄-Alkyl oder R₆ H und R₅ C₁-C₁₅-Alkanoyl oder R₈SO₂- darstellen, gegebenenfalls mit OH substituiert sind,
R₇ H, C₁-C₅-Alkyl, Chlor, Brom bedeutet,
R₈ die gleiche Bedeutung wie R₃ besitzt,
n 2-5 ist sowie, wenn R₄ Wasserstoff bedeutet, deren Salze mit physiologisch verträglichen Basen und deren Cyclodextrinclathrate.

2. Pharmazeutische Präparate gekennzeichnet durch einen Gehalt an Leukotrien-B₄-Derivaten der allgemeinen Formel I gemäß Patentanspruch 1.

3. Verfahren zur Herstellung von Leukotrien-B₄-Derivaten der allgemeinen Formel I, gemäß Patentanspruch 1, dadurch gekennzeichnet, daß man einen Alkohol der Formel II 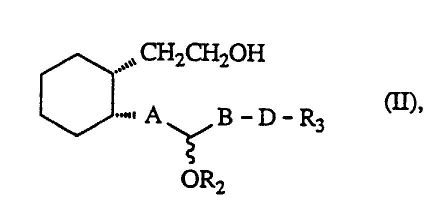 worin A, B, D, R₂ und R₃ die oben angegebene Bedeutung haben, gegebenenfalls nach Schutz freier Hydroxygruppen in R₂ mit einem Alkylhalogenid oder Halogenessigsäurederivat der allgemeinen Formel III,
X-CH₂-R₁ (III)
wobei X Chlor, Brom oder Iod und R₁ -CH₃, CF₃ oder -CH₂OR₉ darstellt, worin R₉ einen leicht abspaltbaren Etherrest bedeutet, in Gegenwart einer Base verethert und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe verseift und/oder reduziert und/oder eine Carboxylgruppe verestert und/oder eine freie Carboxylgruppe in ein Amid überführt oder eine Carboxylgruppe mit einer physiologisch verträglichen Base in ein Salz überführt.

## Claims

1. Leukotriene-B₄ derivatives of general formula I 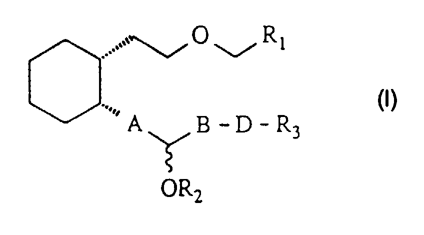 wherein
R₁ represents CH₂OH, CH₃, CF₃, COOR₄ or CONR₅R₆,
R₂ represents H or an organic acid radical having from 1 to 15 carbon atoms,
R₃ represents H, optionally mono- or poly-substituted C₁-C₁₄alkyl or C₃-C₁₀cycloalkyl, a C₆-C₁₀aryl radical that is optionally substituted by one or more substituents selected independently of one another from halogen, phenyl, C₁-C₄alkyl, C₁-C₄alkoxy, fluoromethyl, chloromethyl, trifluoromethyl, carbonyl, carboxy and hydroxy, or a 5- or 6-membered aromatic heterocyclic ring having at least 1 hetero atom,
R₄ represents hydrogen, C₁-C₁₀alkyl, C₃-C₁₀cycloalkyl, a C₆-C₁₀aryl radical that is optionally substituted by from 1 to 3 halogen atoms, phenyl, C₁-C₄alkyl, C₁-C₄alkoxy, fluoromethyl, chloromethyl, trifluoromethyl, carboxyl or by hydroxy, or CH₂-CO-(C₆-C₁₀)aryl or a 5- or 6-membered ring having at least 1 hetero atom,
A represents a trans,trans-CH=CH-CH=CH- group, a -CH₂CH₂-CH=CH- group or a tetramethylene group,
B represents a straight-chain or branched C₁-C₁₀alkylene group which may optionally be substituted by fluorine, or represents the group 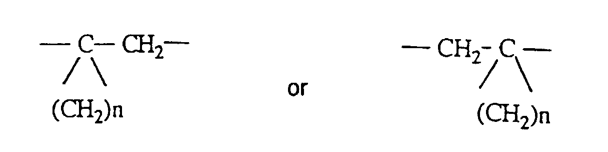
D represents a direct bond, oxygen, sulphur, -C≡C-, -CH=CR₇-, or together with
B can also represent a direct bond,
R₅ and R₆ are identical or different and represent H or C₁-C₄alkyl, or R₆ is H and R₅ is C₁-C₁₅alkanoyl or R₈SO₂-, optionally substituted by OH,
R₇ represents H, C₁-C₅alkyl, chlorine or bromine,
R₈ has the same meanings as R₃,
n is from 2 to 5,
and when R₄ is hydrogen, salts thereof with physiologically tolerable bases and cyclodextrin clathrates thereof.

2. Pharmaceutical preparations characterised in that they comprise leukotriene-B4 derivatives of general formula I according to patent claim 1.

3. Process for the preparation of leukotriene-B₄ derivatives of general formula I according to patent claim 1, characterised in that an alcohol of formula II 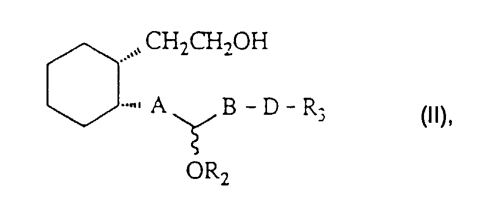 wherein A, B, D, R₂ and R₃ are as defined above, optionally after protection of free hydroxy groups in R₂, is etherified in the presence of a base with an alkyl halide or haloacetic acid derivative of general formual III
X-CH₂-R₁ (III)
wherein X is chlorine, bromine or iodine and R₁ is -CH₃, CF₃ or -CH₂OR₉, wherein R₉ represents a readily removable ether radical, and then optionally, in any sequence, isomers are separated, protected hydroxy groups are freed and/or a free hydroxy group is esterified and/or the 1 -hydroxy group is oxidised to the carboxylic acid and/or double bonds are hydrogenated and/or an esterified carboxy group is hydrolysed and/or reduced and/or a carboxy group is esterified and/or a free carboxy group is converted into an amide or a carboxy group is converted into a salt with a physiologically tolerable base.

## Revendications

1. Dérivés du leucotriène B₄ de formule générale I 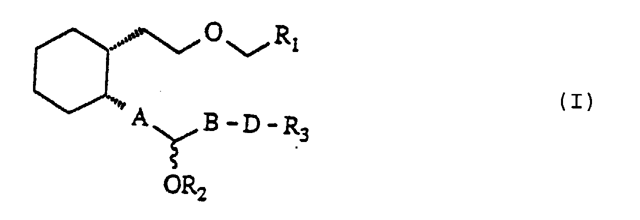 dans laquelle
R₁ représente CH₂OH, CH₃, CF₃, COOR₄, CONR₅R₆,
R₂ représente un atome d'hydrogène ou un reste d'acide organique avec 1 à 15 atomes de carbone,
R₃ représente un atome d'hydrogène, un groupe cycloalkyle en C₃-C₁₀, un groupe alkyle en C₁-C₁₄, substitué une ou plusieurs fois, un reste aryle en C₆-C₁₀ substitué une ou plusieurs fois, éventuellement indépendamment les uns des autres, par un atome d'halogène, des groupes phényle, alkyle en C₁-C₄, alcoxy en C₁-C₄, fluorométhyle, chlorométhyle, trifluorométhyle, carbonyle, carboxyle ou hydroxy ou représente un cycle hétérocyclique aromatique à 5 à 6 chaînons avec au moins un hétéroatome,
R₄ représente un atome d'hydrogène, des groupes alkyle en C₁-C₁₀, cyclalkyle en C₃-C₁₀, un reste aryle en C₆-C₁₀ éventuellement substitué par 1 à 3 atomes d'halogènes, par des groupes phényle, alkyle en C₁-C₄, alcoxy en C₁-C₄, fluorométhyle, chlorométhyle, trifluorométhyle, carboxyle ou hydroxy, CH₂-CO-(aryle C₆-C₁₀) ou un cycle à 5 à 6 chaînons avec au moins un hétéroatome,
A représente un groupe trans,trans-CH=CH-CH=CH, un groupe -CH₂CH₂-CH=CH- ou un groupe tétraméthylène,
B représente un groupe alkylène en C₁-C₁₀ linéaire ou ramifié, qui peut être éventuellement substitué par du fluor, ou le groupe 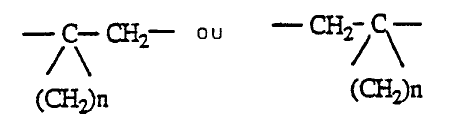
D représente une liaison directe, un atome d'oxygène, un atome de soufre, -C≡C-, -CH=CR₇, ou ensemble avec
B peuvent également former une liaison directe,
R₅ et R₆, identiques ou différents, représentent un atome d'hydrogène ou un groupe alkyle en C₁-C₄ ou R₆ représente un atome d'hydrogène et R₅ représente un alcanoyle en C₁-C₁₅, ou R₈SO₂-, éventuellement substitué par OH,
R₇ représente un atome d'hydrogène, un groupe alkyle en C₁-C₅, un atome de chlore, un atome de brome,
R₈ a la même signification que R₃,
n va de 2 à 5, ainsi que lorsque R₄ est un atome d'hydrogène, leurs sels avec des bases physiologiquement compatibles et leurs clathrates de cyclodextrine.

2. Préparations pharmaceutiques caractérisées par une teneur en dérivés de leucotriène B₄ de formule générale I selon la revendication 1.

3. Procédé pour la préparation des dérivés de leucotriène B₄ de formule générale I, selon la revendication 1, caractérisé en ce que l'on éthérifie un alcool de formule II 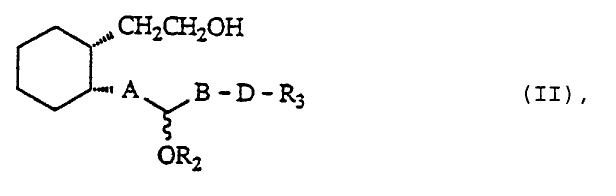 dans laquelle A, B, D, R₂ et R₃ ont la signification donnée ci-dessus, éventuellement après avoir protégé les groupes hydroxy libres dans R₂ avec un halogénure d'alkyle ou un dérivé d'acide halogénoacétique de formule générale III,
X-CH₂-R₁ (III)
dans laquelle X représente un atome de chlore, de brome ou d'iode et R₁ représente -CH₃, CF₃ ou -CH₂OR₉, où R₉ représente un reste éther facilement dissociable, en présence d'une base et éventuellement ensuite en séparant les isomères dans n'importe quel ordre d'opérations, en libérant les groupes hydroxy protégés et/ou en estérifiant un groupe hydroxy libre et/ou en oxydant le groupe 1-hydroxy en acide carboxylique et/ou en hydrogénant les doubles liaisons et/ou en saponifiant un groupe carboxyle estérifié et/ou en réduisant et/ou en estérifiant un groupe carboxyle et/ou en transformant un groupe carboxyle libre en un amide ou en transformant en un sel un groupe carboxyle avec une base physiologiquement compatible.
